# EUROPEAN PATENT SPECIFICATION

(11) **EP 1 140 117 B1**
(45) Date of publication and mention of the grant of the patent: **09.04.2003**
(21) Application number: 99965505.3
(22) Date of filing: 21.12.1999
(51) Int. Cl.: A61K 31/70, A61K 9/20

(54) **ACICLOVIR COMPOSITIONS CONTAINING DIMETHICONE**
DIMETICON ENTHALTENDE ACICLOVIRZUSAMMENSETZUNGEN
COMPOSITIONS D'ACYCLOVIR CONTENANT DU DIMETHICONE

(30) Priority: 23.12.1998 GB 9828620
(43) Date of publication of application: 10.10.2001
(73) Proprietor: GLAXO GROUP LIMITED, Greenford, Middlesex UB6 ONN (GB)
(72) Inventor: CHEEMA, Parbinder Singh GlaxoSmithKline, Barnard Castle County Durham DL12 8DT (GB); GOWER, David GlaxoSmithKline, Stevenage Hertfordshire SG1 2NY (GB); MOUNTER, Andrew David GlaxoSmithKline, Barnard Castle County Durham DL12 8DT (GB); SABEY, Timothy Giles GlaxoSmithKline, Barnard Castle County Durham DL12 8DT (GB)
(74) Representative: Waters, David Martin
(86) International application number: EP9910155
(87) International publication number: WO00038695

(56) References cited:
- EP-A- 0 044 543
- EP-A- 0 394 928
- EP-A- 0 719 540
- EP-A- 0 755 675
- WO-A-93/00905

## Description

This invention relates to a topical pharmaceutical formulation suitable for use in treating virus infections of the skin and mucosa, and in particular it relates to topical formulations containing 9-(2-hydroxyethoxymethyl)guanine, otherwise known as aciclovir, and hereinafter referred to as such.

Aciclovir and pharmaceutically acceptable salts and esters thereof are known to have antiviral activity against various classes viruses both *in vitro* and *in vivo,* see UK patent No. 1 523 865. In particular the compound is active against herpes viruses HSV1 and HSV2 (Herpes Simplex Virus type 1 and type 2) and VZV (varicella zoster virus) which are responsible for a range of infectious diseases in several species especially chicken pox, shingles, genital herpes and herpes labialis (cold sores) in humans and diseases of the skin and mucosa in other animals, including keratitis in rabbits and herpetic encephalitis in mice. Aciclovir has been found to be effective in the treatment of herpes simplex virus and zoster virus infections in humans. Hereinafter references to aciclovir should be understood to include also its pharmaceutically acceptable salts unless the context clearly indicates otherwise.

Aciclovir suffers from the disadvantage that it has a low solubility in water and is almost totally insoluble in hydrophobic solvent systems. It is accordingly difficult to produce a topical formulation containing a sufficient dissolved concentration of active ingredient for it to exert its full effect and also to optimise the flux of the compound into the skin. In addition to ease of release it is also important that any formulation of a pharmaceutically active compound should be stable for long periods of time, should not lose its potency, should not discolour or form insoluble substances or complexes, and also should not be unduly irritating to the skin or mucosa.

European Patent No. 0 044 543 describes oil-in-water topical pharmaceutical formulations of aciclovir wherein the aqueous phase contains at least 30% of a water miscible polyhydric alcohol.

We have now found that oil-in-water topical pharmaceutical formulations of aciclovir comprising dimethicone and at least 25% by weight of a water miscible polyhydric alcohol have particularly advantageous properties. In particular, such formulations exhibit surprisingly low irritancy together with good physical stability and ease of application.

A first aspect of the invention provides a topical pharmaceutical formulation comprising aciclovir, dimethicone and at least 30% w/w of a water miscible polyhydric alcohol; wherein the water miscible polyhydric alcohol is propylene glycol, butane 1,3-diol or diethylene glycol monoethyl ether, and wherein the dimethicone is present in an amount of from 0.1% to 10% by weight of the formulation.

Preferably, the topical pharmaceutical formulation is an oil-in-water formulation.

A second aspect of the invention provides an oil-in-water topical pharmaceutical formulation comprising a continuous aqueous phase and, dispersed therein, an oil phase, the formulation comprising water, solubilised aciclovir, dimethicone and at least 30% w/w of a water miscible polyhydric alcohol; wherein the water miscible polyhydric alcohol is propylene glycol, butane 1,3-diol or diethylene glycol monoethyl ether, and wherein the dimethicone is present in an amount of from 0.1% to 10% by weight of the formulation.

A third aspect of the present invention provides a topical pharmaceutical formulation, in particular an oil-in-water topical pharmaceutical formulation, comprising water, aciclovir, dimethicone and at least 30% of a water miscible polyhydric alcohol by weight of the formulation; wherein the formulation contains a maximum of 50% water by weight of the formulation, wherein the water miscible polyhydric alcohol is propylene glycol, butane 1,3-diol or diethylene glycol monoethyl ether, and wherein the dimethicone is present in an amount of from 0.1% to 10% by weight of the formulation.

In a topical pharmaceutical formulation according to the invention, the polyhydric alcohol is preferably present in a or the aqueous phase in an amount up to about 50% by weight.

Preferably the formulation of the invention contains at least 30% by weight of the water miscible polyhydric alcohol and a maximum of 50% water by weight of the formulation.

Such a topical formulation may contain 0.075% to 10% w/w aciclovir or a salt thereof, from 30% to 50% w/w of the water miscible polyhydric alcohol, from 15% to 50% w/w water, dimethicone and an oil phase.

In a preferred aspect, the formulation comprises from 0.5% to 10% w/w aciclovir, from 30% to 45% w/w of the water miscible polyhydric alcohol and from 20% to 40% w/w water together with an oil phase and dimethicone, whilst the most preferred formulation comprises from 1% to 5% w/w aciclovir, from 30% to 40% w/w of the water miscible polyhydric alcohol, from 25% to 40% w/w water, an oil phase and dimethicone.

The dimethicone may be present in an amount sufficient to significantly decrease the irritancy of the composition. The dimethicone is present in an amount from 0.1% to 10% by weight of the formulation, preferably from 0.1% to 5.0% by weight. In the most preferred formulations, the dimethicone will be present in an amount from 0.5% to 1.5% by weight of the formulation.

Dimethicone, also known as polydimethylsiloxane, is a silicone oil consisting of a mixture of fully methylated linear siloxane polymers end-blocked with trimethyl siloxy units. It finds use as an emollient or glidant in cream based pharmaceutical formulations.

A polyhydric alcohol is an alcohol having two or more hydroxyl groups. Water miscible polyhydric alcohols suitable for incorporation into the formulations of the present invention are propylene glycol, butane 1,3-diol, and diethylene glycol monoethyl ether, the preferred alcohol being propylene glycol. The formulations of the invention may comprise from 25% to 50% propylene glycol, for example from 25% to 45% by weight, desirably from 35% to 45% by weight, particularly 40% by weight of the formulation.

The formulation of the invention may be an oil-in-water topical pharmaceutical formulation wherein the polyhydric alcohol is propylene glycol, for example wherein the propylene glycol is present in an amount of from 30% to 45% w/w or from 30% to 40% w/w.

The oil phase of the emulsions of this invention may be constituted from known ingredients in a known manner. While the oil phase may comprise merely an emulsifier (otherwise known as an emulgent), it is desirably comprised of a mixture of at least one emulsifier with a fat or an oil or with both a fat and an oil. Preferably, as explained in more detail below, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so called emulsifying ointment base which forms the oil dispersed phase of the emulsions.

Emulgents and emulsion stabilisers suitable for use in the formulation of the present invention include cetyl alcohol, sodium lauryl sulphate, stearyl alcohol and polyoxyethylene alkyl ethers, such as polyoxyl stearyl ethers, for example steareth 2 and steareth 21.

The choice of suitable oils or fats for the formulation is based on achieving the desired cosmetic properties, since the solubility of aciclovir in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dialkyl esters such as diisopropyl adipate, isocetyl stearate, propylene glycol diester of coconut fatty acids, isopropyl myristate, decyl oleate, isopropyl palmitate, butyl stearate, 2-ethylhexyl palmitate or a mixed ester of 2-ethyl hexanoic acid with a blend of cetyl or stearyl alcohols known as Crodamol CAP may be used, the last three being the preferred esters. These may be used singly or in combination depending on the properties required. Alternatively, high melting point lipids such as white soft paraffin and/or liquid paraffin or other mineral oils can be used.

A preferred formulation according to the invention comprises 0.5-1.5% w/w dimethicone; approximately 5% w/w aciclovir; 5-10% w/w cetostearyl alcohol; 35-45% w/w propylene glycol; 2-10% w/w mineral oil; 10-15% w/w white petrolatum; 0.5-1% w/w sodium lauryl sulphate; 0.5-2% w/w poloxamer 407 and purified water to 100% w/w.

The formulations of the invention may, if desired, include one or more pharmaceutically acceptable preservatives. However, we have found that the use of preservatives is not essential in the formulations of the invention, which finding represents an advantage of the said formulations.

The formulations of the invention may, if desired, include one or more pharmaceutically acceptable excipients such as colours, fragrances, and other excipients known to those skilled in the art of topical formulation and cosmetic cream manufacture. All such excipients must be "pharmaceutically acceptable" in the sense of being compatible with the other ingredients of the formulation and not injurious to the patient.

A formulation according to the present invention can be used in a method of treating a viral infection caused by a member of the herpes family of viruses, the method comprising topical administration of a pharmaceutically effective amount of the formulation. The present invention provides the use of aciclovir, dimethicone and at least 30% w/w of a water miscible polyhydric alcohol in the preparation of a topical medicament for the treatment of herpes family viral infections; wherein the water miscible polyhydric alcohol is propylene glycol, butane 1,3-diol or diethylene glycol monoethyl ether, and wherein the dimethicone is present in an amount of from 0.1% to 10% by weight of the medicament.

The present invention also provides the use of dimethicone in decreasing the irritancy of topical oil-in-water formulations of aciclovir comprising at least 30% w/w of a water miscible polyhydric alcohol; wherein the water miscible polyhydric alcohol is propylene glycol, butane 1,3-diol or diethylene glycol monoethyl ether, and wherein the dimethicone is present in an amount of from 0.1% to 10% by weight of the formulation.

The present invention further provides a method for the preparation of a topical pharmaceutical formulation, as hereinbefore defined, which comprises mixing the combination of aciclovir, the water miscible polyhydric alcohol and water with an oil phase and the dimethicone to form a cream emulsion.

The manner of formulating the emulsion will of course vary according to the amount and nature of the constituents, but nevertheless follows known techniques in emulsion technology (see the Pharmaceutical Codex, London, the Pharmaceutical Press, 1979). For example the aciclovir and water miscible polyhydric alcohol may initially be incorporated wholly in the aqueous portion where they may form a solution, or a mixed solution/suspension, and be then emulsified with an ointment base and the dimethicone. Alternatively where high concentrations of aciclovir are being used, a part of the aqueous portion may be formulated with the ointment base as an emulsion, and the balance of the water miscible polyhydric alcohol and aciclovir added to and dispersed into the emulsion. In another technique the aciclovir may be included in the emulsifying ointment prior to emulsification with the aqueous portion. In using these procedures, it is preferable to heat the aqueous portion and the ointment base to about 40 to 80°C, preferably 50 to 70°C, prior to emulsification which may be achieved by vigorous agitation using for example a standard laboratory mixer. Finer dispersions of the oil phase may be obtained by homogenising or milling in a colloidal mill.

A topical formulation of the present invention may be used in the treatment or prevention of viral infections caused for example by Herpes zoster, Herpes varicella and Herpes simplex types I and 2, which cause diseases such as shingles, chicken pox, cold sores and genital herpes. The formulation should desirably be applied to the affected area of skin from 1 to 6 times daily, preferably from 3 to 5 times.

The following examples illustrate the invention and are not intended as a limitation thereof.

### Example 1

| Ingredient | % w/w |
|---|---|
| TRANSCUTOL™ | 40.0 |
| aciclovir | 5.0 |
| stearyl alcohol | 5.0 |
| cetyl alcohol | 4.0 |
| light mineral oil | 10.2 |
| brij 721 | 2.5 |
| brij 72 | 2.3 |
| dimethicone 20 | 1.5 |
| Purified water | to 100 |

The oil phase comprising stearyl alcohol, cetyl alcohol, light mineral oil, brij 72 and brij 721 is heated to 70-75°C with mixing and the dimethicone is added. Purified water is heated to 65-70°C and added to the oil phase, maintaining the temperature at 70-75°C, with mixing to form an emulsion. The mixture is maintained at a temperature of 70-75°C for approximately 5 minutes. TRANSCUTOL™ is weighed into an appropriate container and aciclovir added with mixing to form a suspension. The aciclovir suspension is added to the emulsion, rinsing in with a small amount of purified water. The emulsion is homogenised for approximately 5 minutes, then made up to final batch weight with purified water. The resulting cream is cooled to ambient temperature (approximately 30°C) with continuous mixing before the cream is filled into suitable tubes which are then sealed.

### Example 2

| Ingredient | % w/w |
|---|---|
| aciclovir | 5.0 |
| mineral oil | 5.0 |
| white petrolatum | 11.5 |
| sodium lauryl sulphate | 0.75 |
| cetostearyl alcohol | 6.75 |
| propylene glycol | 40.0 |
| poloxamer 407 | 1.0 |
| dimethicone | 1.0 |
| purified water | to 100 |

In vessel 1, poloxamer 407 was dissolved in cold purified water. Then the propylene glycol was added and mixed to form the aqueous phase. A small portion of the aqueous phase was removed and added to the aciclovir powder in a second pot, and an aciclovir dispersion formed. The remaining aqueous phase in vessel 1 was heated to 70°C - 80°C. The emulsifying wax was prepared in vessel 3 by heating together the cetostearyl alcohol, sodium lauryl sulphate and a small quantity of purified water at 115°C until frothing ceased. The prepared emulsifying wax was melted together with the white soft petrolatum, the dimethicone and the mineral oil and the mixture heated to 73-77°C. The hot aqueous phase was then added to vessel 3 and the mixture emulsified. The emulsion was cooled to 45 - 55°C. The aciclovir suspension was added and mixed in well. The cream was cooled to below 40°C and transferred to a holding vessel, before being filled into suitable tubes which were then sealed.

### Example 3

The formulation described in Example 1 may alternatively be prepared by the following modified procedure.

The oil phase is weighed and heated to 73-77°C and the dimethicone is added, with continuous slow mixing. Purified water is heated to 65-70°C. The purified water is added with propeller agitation to the suspension of aciclovir in TRANSCUTOL™. The resulting aqueous mixture is heated to 65-70°C. Whilst maintaining the temperature of the oil phase at 70-75°C, the aqueous phase is slowly added with sweep agitation for at least 5 minutes. The aqueous phase container is rinsed with purified water and the rinsings added to the main batch. The temperature of the batch is maintained at 70-75°C and the batch is homogenized for at least 5 minutes. The batch is cooled to 30-35°C with continuous sweep agitation and purified water added to adjust to final batch weight. The batch is mixed until uniform and cooled to 30°C before the cream is filled into suitable tubes which are then sealed.

### Example 4

5% aciclovir cream (marketed under the trademark ZOVIRAX® by Glaxo Wellcome, Glaxo Wellcome House, Berkeley Avenue Greenford, Middlesex, UB6 0NN) consists of aciclovir solubilised in the aqueous phase of the oil-in-water cream base known as "MAC-P".

### MAC-P Cream Base

| | %w/w |
|---|---|
| Propylene glycol | 40.00 |
| Cetostearyl alcohol | 6.75 |
| Sodium lauryl sulphate | 0.75 |
| Poloxamer 407 (Pluronic F127 ™) | 1.00 |
| White soft paraffin (petrolatum) | 12.50 |
| Liquid paraffin (mineral oil) | 5.00 |
| Purified water | to 100.00 |

This formulation is very similar to that of Example 2 and is made in the same way; for MAC-P cream base aciclovir is omitted, for ZOVIRAX® cream, 5% w/w aciclovir is included. MAC-P cream, with or without aciclovir, may also be formulated with dimethicone, which is added to the oil phase which is held at 73-77°C.

### Experimental Data

The guinea pig model of cutaneous herpes simplex virus (HSV) infection was used to compare the ZOVIRAX® (5% w/w aciclovir in MAC-P cream) formulation with MAC-P plus 1% dimethicone and with MAC-P plus 1% dimethicone and 5% aciclovir (made as set out above in examples 2 and 4).

### Guinea Pig Model:

The guinea pig model of HSV infection is characterised by a self-limiting cutaneous disease which provides a useful model in the evaluation and development of topical HSV therapies. The disease produced by experimentally infecting guinea pigs with HSV mimics the human infection in clinical appearance, disease progression and duration of lesions.

### Infection Procedure:

Female hairless guinea pigs (lbm:GOHI-hr) weighing between 550g and 600g (BRL Biological Research Laboratories Limited, Wolferstrasse 4, CH-4414 Fullinsdorf, Switzerland), sedated with an intramuscular injection of Hypnorm 0.6ml/kg (Janssen Animal Health, High Wycombe, UK), were inoculated on the left flank at six discrete sites. This infection procedure involved the light scarification of the skin surface through a 20µl drop of viral suspension (final virus titre of 3x10⁵ pfu/20µl) at each of the sites, using a disposable multi-tine apparatus with 6 x 2mm tines (Bignell Surgical Supplies, Littlehampton, West Sussex, UK).

### Treatment Regimen:

A total of 25 animals were used in this study. Seven animals were allocated to the untreated control group and six to each of three treatment groups:
1. No treatment (Virus control)
2. 1% Dimethicone in MAC-P
3. 5% aciclovir in MAC-P (ZOVIRAX®)
4. 1% Dimethicone + 5% aciclovir in MAC-P

Therapy consisted of 1ml (approx. 1g) of test cream, delivered from a 5ml syringe, spread evenly over the infected flank twice daily (8:00am and 5:00pm). Treatment of infected animals commenced 18 hours post infection and continued for three days, with each animal receiving a total of six applications.

### Disease Progression:

During the normal course of infection, inoculation sites become erythematous and oedematous by day 1 post infection (p.i.), the extent of which increases over subsequent days. The emergence of papules, which subsequently develop into pustules, is generally observed by 2 to 3 days p.i.. the size and number of pustules increases as the infection progresses and, if in juxtaposition, they will eventually coalesce. The peak of infection is generally observed by day 6-7 p.i. when coalesced lesions begin to crust over.

### Determination Of Antiviral Effect:

In order to determine disease progression, a daily assessment of each animal was made. This involved scoring each of the infection sites according to the criteria set out below:
0.0 = no signs of visible infection
0.5 = very early papules (not raised) slight erythema
1.0 = papules and erythema
2.0 = 0-5 pustules and erythema
3.0 = 6+ pustules and erythema
4.0 = coalescence of pustules
5.0 = crusting/scabbing of pustules

The first assessment was carried out immediately prior to the commencement of treatment and was repeated on days 2,3,4 and 8 post infection. As the method of assessment is based on the natural progression of disease in guinea pigs, a reduction in for treated animals over untreated animals indicates an antiviral effect. The daily scores for each flank were totaled and the average score per flank per day calculated. The areas under the curve (AUCs) for all treatment groups and the control group were calculated and subjected to statistical analysis to determine significance.

### Determination Of Erythema

A visual inspection made of each animal at the time of treatment and the degree of erythema on each flank was noted. This treatment-related erythema was assigned to one of five categories:
1 no erythema
2 slight erythema
3 moderate erythema
4 substantial erythema
5 severe erythema

### Results:

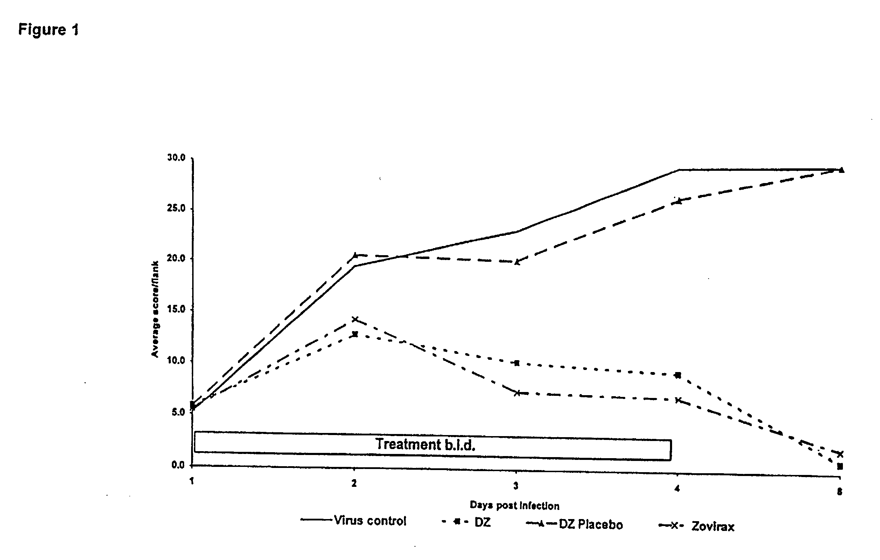

Results for antiviral activity are shown in tables 1a-d, table 2 and fig. 1 and for erythema in table 3.

### Antiviral

All animals in the control group reached the maximum possible lesion score by day 8 p.i.. Animals treated with placebo (group 2: 1% Dimethicone in MAC-P) developed a disease profile closely resembling the control group, showing that the 1% dimethicone cream offers no antiviral benefit. Treatment with both Zovirax® and the 1% Dimethicone + 5% aciclovir in MAC-P creams resulted in a reduction in lesion development after only two applications of therapy. In both cases, this reduction continued with further applications of treatment, resulting in few signs of clinical infection remaining visible by day 8 post infection.

**Table 1a**

| **Individual lesion scores - Virus control group** | | | | | | | | | | | | | | | |
|---|---|---|---|---|---|---|---|---|---|---|---|---|---|---|---|
| | **Day 1 post infection** | | | **Day 2 post infection** | | | **Day 3 post infection** | | | **Day 4 post infection** | | | **Day 8 post infection** | | |
| Virus control NM | 1.0 | 1.0 | 1.0 | 3.0 | 3.0 | 3.0 | 4.0 | 4.0 | 4.0 | 5.0 | 5.0 | 4.0 | 5.0 | 5.0 | 5.0 |
| | 1.0 | 1.0 | 1.0 | 3.0 | 3.0 | 3.0 | 4.0 | 4.0 | 4.0 | 5.0 | 5.0 | 5.0 | 5.0 | 5.0 | 5.0 |
| Virus Control 1F | 1.0 | 1.0 | 1.0 | 3.0 | 3.0 | 3.0 | 4.0 | 4.0 | 4.0 | 5.0 | 5.0 | 5.0 | 5.0 | 5.0 | 5.0 |
| | 1.0 | 1.0 | 0.5 | 3.0 | 3.0 | 3.0 | 4.0 | 4.0 | 4.0 | 5.0 | 5.0 | 5.0 | 5.0 | 5.0 | 5.0 |
| Virus Control 1B | 1.0 | 1.0 | 1.0 | 4.0 | 4.0 | 3.0 | 4.0 | 4.0 | 4.0 | 5.0 | 4.0 | 5.0 | 5.0 | 5.0 | 5.0 |
| | 1.0 | 1.0 | 1.0 | 4.0 | 4.0 | 4.0 | 4.0 | 4.0 | 4.0 | 5.0 | 5.0 | 5.0 | 5.0 | 5.0 | 5.0 |
| Virus Control 2F | 1.0 | 1.0 | 1.0 | 4.0 | 4.0 | 4.0 | 3.0 | 4.0 | 4.0 | 5.0 | 5.0 | 5.0 | 5.0 | 5.0 | 5.0 |
| | 1.0 | 1.0 | 1.0 | 4.0 | 4.0 | 4.0 | 4.0 | 4.0 | 4.0 | 5.0 | 5.0 | 5.0 | 5.0 | 5.0 | 5.0 |
| Virus Control 2B | 1.0 | 1.0 | 0.5 | 3.0 | 4.0 | 30 | 3.0 | 4.0 | 3.0 | 5.0 | 5.0 | 5.0 | 5.0 | 5.0 | 5.0 |
| | 1.0 | 1.0 | 0.5 | 3.0 | 3.0 | 3.0 | 4.0 | 4.0 | 4.0 | 5.0 | 5.0 | 5.0 | 5.0 | 5.0 | 5.0 |
| Virus Control 1F1B | 1.0 | 1.0 | 0.5 | 3.0 | 3.0 | 3.0 | 4.0 | 4.0 | 4.0 | 5.0 | 5.0 | 5.0 | 5.0 | 5.0 | 5.0 |
| | 1.0 | 1.0 | 0.5 | 3.0 | 4.0 | 3.0 | 5.0 | 4.0 | 4.0 | 5.0 | 5.0 | 5.0 | 5.0 | 5.0 | 5.0 |
| Virus Control 2F1B | 0.5 | 0.5 | 0.5 | 3.0 | 3.0 | 3.0 | 3.0 | 3.0 | 4.0 | 5.0 | 5.0 | 5.0 | 5.0 | 5.0 | 5.0 |
| | 0.5 | 0.5 | 0.5 | 3.0 | 2.0 | 2.0 | 3.0 | 4.0 | 4.0 | 5.0 | 5.0 | 5.0 | 5.0 | 5.0 | 5.0 |

**Table 1b**

| **Individual lesion scores -1% Dimethicone / Zovirax placebo treated group** | | | | | | | | | | | | | | | |
|---|---|---|---|---|---|---|---|---|---|---|---|---|---|---|---|
| | **Day 1 post infection** | | | **Day 2 post infection** | | | **Day 3 post infection** | | | **Day 4 post infection** | | | **Day 8 post infection** | | |
| DZ Placebo NM | 1.0 | 1.0 | 1.0 | 3.0 | 3.0 | 3.0 | 4.0 | 3.0 | 4.0 | 4.0 | 3.0 | 3.0 | 5.0 | 5.0 | 5.0 |
| | 1.0 | 1.0 | 1.0 | 3.0 | 3.0 | 3.0 | 3.0 | 3.0 | 3.0 | 3.0 | 3.0 | 3.0 | 5.0 | 5.0 | 5.0 |
| DZ Placebo 1F | 1.0 | 1.0 | 1.0 | 4.0 | 4.0 | 3.0 | 4.0 | 4.0 | 4.0 | 5.0 | 5.0 | 5.0 | 5.0 | 5.0 | 5.0 |
| | 1.0 | 1.0 | 1.0 | 3.0 | 3.0 | 3.0 | 3.0 | 4.0 | 3.0 | 5.0 | 5.0 | 5.0 | 5.0 | 5.0 | 5.0 |
| DZ Placebo 1B | 1.0 | 1.0 | 1.0 | 4.0 | 4.0 | 4.0 | 4.0 | 4.0 | 4.0 | 5.0 | 5.0 | 5.0 | 5.0 | 5.0 | 5.0 |
| | 1.0 | 1.0 | 1.0 | 4.0 | 4.0 | 4.0 | 4.0 | 4.0 | 4.0 | 5.0 | 5.0 | 5.0 | 5.0 | 5.0 | 5.0 |
| DZ Placebo 2F | 1.0 | 1.0 | 1.0 | 4.0 | 4.0 | 4.0 | 4.0 | 4.0 | 4.0 | 4.0 | 4.0 | 4.0 | 5.0 | 5.0 | 5.0 |
| | 1.0 | 1.0 | 1.0 | 2.0 | 2.0 | 3.0 | 2.0 | 3.0 | 3.0 | 3.0 | 3.0 | 3.0 | 5.0 | 5.0 | 5.0 |
| DZ Placebo 2B | 1.0 | 1.0 | 0.5 | 3.0 | 4.0 | 4.0 | 20. | 2.0 | 2.0 | 5.0 | 5.0 | 5.0 | 5.0 | 5.0 | 5.0 |
| | 1.0 | 1.0 | 0.5 | 3.0 | 4.0 | 3.0 | 3.0 | 3.0 | 3.0 | 5.0 | 5.0 | 5.0 | 5.0 | 5.0 | 5.0 |
| DZ Placebo 1F1B | 1.0 | 1.0 | 1.0 | 3.0 | 4.0 | 4.0 | 4.0 | 4.0 | 4.0 | 5.0 | 5.0 | 5.0 | 5.0 | 5.0 | 5.0 |
| | 1.0 | 1.0 | 0.5 | 3.0 | 4.0 | 4.0 | 3.0 | 3.0 | 3.0 | 5.0 | 5.0 | 5.0 | 5.0 | 5.0 | 5.0 |

**Table 1c**

| **Individual lesion scores - Zovirax treated group** | | | | | | | | | | | | | | | |
|---|---|---|---|---|---|---|---|---|---|---|---|---|---|---|---|
| | **Day 1 post infection** | | | **Day 2 post infection** | | | **Day 3 post infection** | | | **Day 4 post infection** | | | **Day 8 post infection** | | |
| Zovirax NM | 1.0 | 1.0 | 0.5 | 3.0 | 3.0 | 3.0 | 1.0 | 2.0 | 1.0 | 1.0 | 2.0 | 1.0 | 0.0 | 0.0 | 0.0 |
| | 1.0 | 0.5 | 0.5 | 2.0 | 1.0 | 1.0 | 2.0 | 1.0 | 1.0 | 1.0 | 1.0 | 1.0 | 0.0 | 0.0 | 0.0 |
| Zovirax 1F | 1.0 | 1.0 | 1.0 | 3.0 | 3.0 | 3.0 | 1.0 | 1.0 | 2.0 | 1.0 | 2.0 | 1.0 | 1.0 | 1.0 | 1.0 |
| | 1.0 | 0.5 | 1.0 | 3.0 | 2.0 | 3.0 | 1.0 | 1.0 | 1.0 | 2.0 | 1.0 | 1.0 | 1.0 | 1.0 | 1.0 |
| Zovirax 1B | 1.0 | 1.0 | 1.0 | 3.0 | 3.0 | 3.0 | 2.0 | 1.0 | 1.0 | 1.0 | 1.0 | 2.0 | 1.0 | 1.0 | 1.0 |
| | 1.0 | 1.0 | 1.0 | 2.0 | 2.0 | 2.0 | 2.0 | 2.0 | 1.0 | 1.0 | 2.0 | 1.0 | 0.0 | 0.0 | 0.0 |
| Zovirax 2F | 1.0 | 1.0 | 1.0 | 3.0 | 3.0 | 3.0 | 1.0 | 2.0 | 2.0 | 1.0 | 2.0 | 2.0 | 0.0 | 1.0 | 1.0 |
| | 1.0 | 1.0 | 0.5 | 2.0 | 2.0 | 2.0 | 1.0 | 1.0 | 1.0 | 1.0 | 1.0 | 0.5 | 0.0 | 0.0 | 0.0 |
| Zovirax 2B | 1.0 | 1.0 | 1.0 | 3.0 | 3.0 | 3.0 | 1.0 | 1.0 | 2.0 | 1.0 | 1.0 | 1.0 | 0.0 | 0.0 | 0.0 |
| | 1.0 | 1.0 | 1.0 | 2.0 | 1.0 | 1.0 | 1.0 | 1.0 | 1.0 | 1.0 | 1.0 | 1.0 | 1.0 | 1.0 | 0.0 |
| Zovirax 1F1B | 1.0 | 1.0 | 1.0 | 3.0 | 3.0 | 2.0 | 1.0 | 1.0 | 1.0 | 1.0 | 1.0 | 1.0 | 0.0 | 0.0 | 0.0 |
| | 1.0 | 1.0 | 0.5 | 2.0 | 2.0 | 1.0 | 1.0 | 1.0 | 1.0 | 1.0 | 1.0 | 1.0 | 0.0 | 0.0 | 0.0 |

**Table 1d**

| **Individual lesion scores -1% Dimethicone / Zovirax (DZ) treated group** | | | | | | | | | | | | | | | |
|---|---|---|---|---|---|---|---|---|---|---|---|---|---|---|---|
| | **Day 1 post infection** | | | **Day 2 post infection** | | | **Day 3 post infection** | | | **Day 4 post infection** | | | **Day 8 post Infection** | | |
| DZ NM | 1.0 | 1.0 | 1.0 | 3.0 | 2.0 | 2.0 | 2.0 | 1.0 | 1.0 | 2.0 | 1.0 | 2.0 | 0.0 | 0.0 | 0.0 |
| | 1.0 | 1.0 | 1.0 | 2.0 | 2.0 | 2.0 | 1.0 | 1.0 | 1.0 | 1.0 | 2.0 | 1.0 | 0.0 | 0.0 | 0.0 |
| DZ 1F | 1.0 | 1.0 | 1.0 | 2.0 | 2.0 | 2.0 | 2.0 | 1.0 | 1.0 | 2.0 | 1.0 | 2.0 | 0.0 | 0.0 | 0.0 |
| | 1.0 | 1.0 | 1.0 | 2.0 | 2.0 | 2.0 | 1.0 | 2.0 | 1.0 | 1.0 | 2.0 | 2.0 | 0.0 | 0.0 | 0.0 |
| DZ 1B | 1.0 | 1.0 | 1.0 | 3.0 | 3.0 | 3.0 | 2.0 | 2.0 | 3.0 | 1.0 | 2.0 | 2.0 | 0.0 | 0.0 | 0.0 |
| | 1.0 | 1.0 | 1.0 | 2.0 | 3.0 | 2.0 | 2.0 | 2.0 | 2.0 | 1.0 | 1.0 | 2.0 | 0.0 | 0.0 | 0.0 |
| DZ 2F | 1.0 | 1.0 | 1.0 | 2.0 | 2.0 | 2.0 | 2.0 | 2.0 | 2.0 | 2.0 | 1.0 | 2.0 | 0.0 | 0.0 | 0.0 |
| | 1.0 | 1.0 | 1.0 | 1.0 | 1.0 | 1.0 | 1.0 | 1.0 | 1.0 | 2.0 | 1.0 | 1.0 | 1.0 | 0.0 | 1.0 |
| DZ 2B | 1.0 | 1.0 | 1.0 | 3.0 | 3.0 | 3.0 | 2.0. | 3.0 | 2.0 | 2.0 | 2.0 | 2.0 | 1.0 | 0.0 | 0.0 |
| | 1.0 | 0.5 | 0.5 | 1.0 | 1.0 | 2.0 | 1.0 | 1.0 | 1.0 | 2.0 | 2.0 | 0.0 | 0.0 | 1.0 | 0.0 |
| DZ 1F1B | 1.0 | 1.0 | 1.0 | 3.0 | 2.0 | 3.0 | 3.0 | 3.0 | 3.0 | 1.0 | 1.0 | 2.0 | 1.0 | 0.0 | 1.0 |
| | 1.0 | 1.0 | 1.0 | 2.0 | 2.0 | 2.0 | 2.0 | 2.0 | 2.0 | 1.0 | 1.0 | 2.0 | 0.0 | 0.0 | 0.0 |

**Table 2**

| **Average score per flank** | | | | | |
|---|---|---|---|---|---|
| | **Day 1 post Infection** | **Day 2 post infection** | **Day 3 post infection** | **Day 4 post infection** | **Day 8 post infection** |
| Virus Control | 5.2 | 19.6 | 23.3 | 29.7 | 30 |
| DZ Placebo | 5.8 | 20.7 | 20.3 | 26.7 | 30 |
| Zovirax | 5.5 | 14.3 | 7.5 | 7.1 | 2.2 |
| DZ | 5.8 | 12.8 | 10.3 | 10 | 1 |

Statistical analysis of the data generated suggested a highly significant difference between the control and placebo treated groups and the Zovirax® and 1% Dimethicone + 5% aciclovir treated groups (p<0.001). There was, however, no evidence of a significant difference between the Zovirax® and the 1% Dimethicone + 5% aciclovir in MAC-P groups (p=0.34).

### Erythema

As can be seen from table 3, substantial erythema was noted following treatment with Zovirax®, whereas only slight erythema was observed following treatment with the 1% Dimethicone + 5% aciclovir in MAC-P cream (DZ).

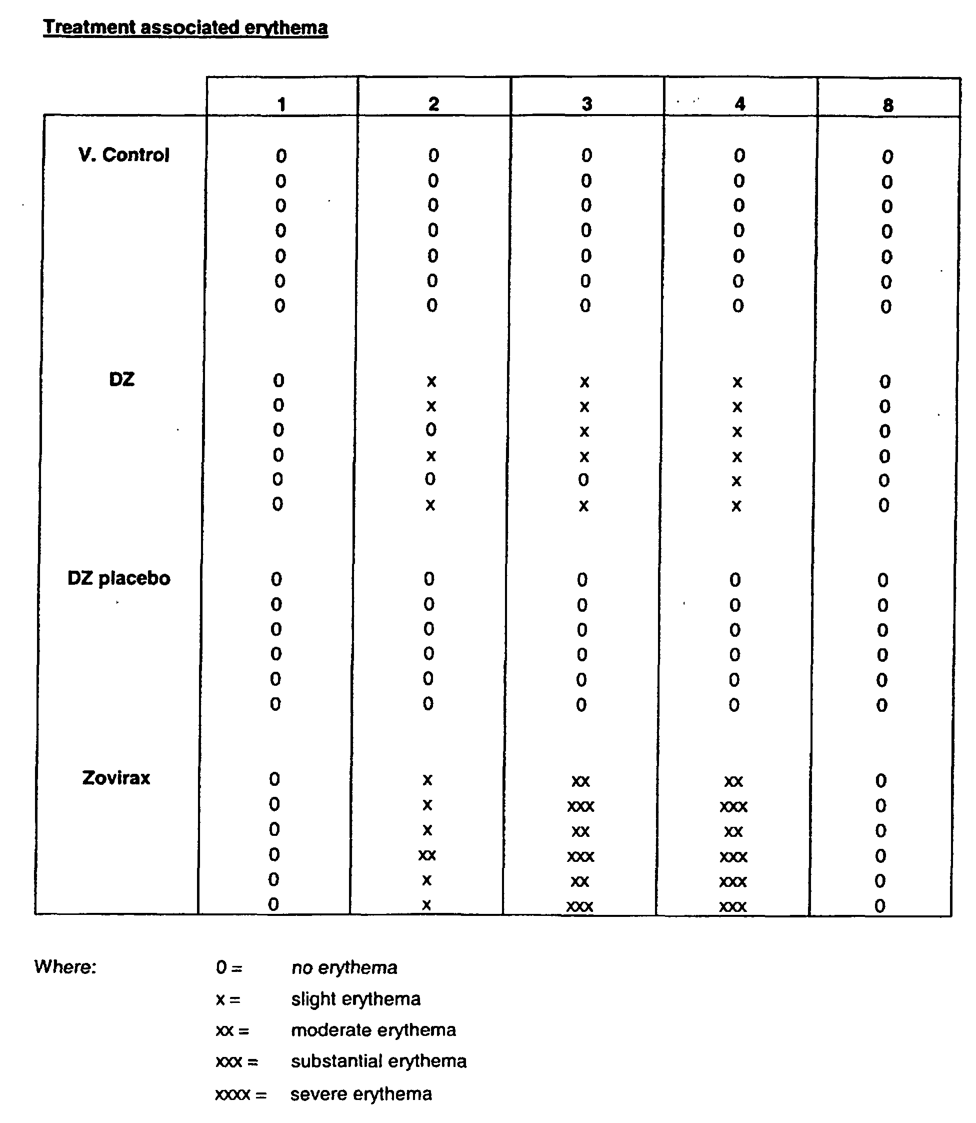

### Discussion:

The side effect of treatment-related erythema has frequently been observed in animals treated with Zovirax®, which can lead to cracking and desquamation of skin. From the observations made in this study it is apparent that the addition of 1% Dimethicone to the formulation results in a significant reduction of the level of treatment associated erythema, whilst the high level of antiviral activity is maintained.

## Claims

1. A topical pharmaceutical formulation comprising aciclovir, dimethicone and at least 30% w/w of a water miscible polyhydric alcohol;
wherein the water miscible polyhydric alcohol is propylene glycol, butane 1,3-diol or diethylene glycol monoethyl ether, and wherein the dimethicone is present in an amount of from 0.1% to 10% by weight of the formulation.

2. A topical pharmaceutical formulation according to claim 1 which is an oil-in-water formulation.

3. An oil-in-water topical pharmaceutical formulation comprising a continuous aqueous phase and, dispersed therein, an oil phase, the formulation comprising water, solubilised aciclovir, dimethicone and at least 30% w/w of a water miscible polyhydric alcohol;
wherein the water miscible polyhydric alcohol is propylene glycol, butane 1,3-diol or diethylene glycol monoethyl ether, and wherein the dimethicone is present in an amount of from 0.1% to 10% by weight of the formulation.

4. A topical pharmaceutical formulation according to any one of claims 1 to 3 containing at least 30% by weight of the water miscible polyhydric alcohol and a maximum of 50% water by weight of the formulation.

5. A topical pharmaceutical formulation comprising water, aciclovir, dimethicone and at least 30% of a water miscible polyhydric alcohol by weight of the formulation;
wherein the formulation contains a maximum of 50% water by weight of the formulation, wherein the water miscible polyhydric alcohol is propylene glycol, butane 1,3-diol or diethylene glycol monoethyl ether, and wherein the dimethicone is present in an amount of from 0.1% to 10% by weight of the formulation.

6. A topical pharmaceutical formulation according to claim 5 which is an oil-in-water topical pharmaceutical formulation.

7. A topical pharmaceutical formulation according to any of claims 1 to 6 wherein the polyhydric alcohol is present in a or the aqueous phase in an amount up to about 50% by weight.

8. A topical pharmaceutical formulation according to any of claims 1 to 7 containing 0.075% to 10% w/w aciclovir or a salt thereof, from 30% to 50% w/w of the water miscible polyhydric alcohol, from 15% to 50% w/w water, dimethicone and an oil phase.

9. A topical pharmaceutical formulation according to claim 8 comprising from 0.5% to 10% w/w aciclovir, from 30% to 45% w/w of the water miscible polyhydric alcohol and from 20% to 40% w/w water together with an oil phase and dimethicone.

10. A topical pharmaceutical formulation according to any of claims 1 to 9, wherein the water miscible polyhydric alcohol is propylene glycol or diethylene glycol monoethyl ether.

11. A topical pharmaceutical formulation according to any of claims 1 to 9, wherein the water miscible polyhydric alcohol is propylene glycol.

12. A topical pharmaceutical formulation according to claim 11 comprising from 35% to 45% propylene glycol by weight of the formulation.

13. An oil-in-water topical pharmaceutical formulation according to any one of claims 2 to 9, wherein the polyhydric alcohol is propylene glycol.

14. An oil-in-water topical pharmaceutical formulation according to claim 13 wherein the propylene glycol is present in an amount of from 30% to 45% w/w.

15. An oil-in-water topical pharmaceutical formulation according to claim 13 wherein the propylene glycol is present in an amount of from 30% to 40% w/w.

16. A topical pharmaceutical formulation according to any preceding claim wherein the dimethicone is present in an amount sufficient to significantly decrease the irritancy of the formulation.

17. A topical pharmaceutical formulation according to any preceding claim wherein the dimethicone is present in an amount of from 0.5% to 10% by weight of the formulation.

18. A topical pharmaceutical formulation according to any preceding claim wherein the dimethicone is present in an amount of from 0.1% to 5.0% by weight of the formulation.

19. A topical pharmaceutical formulation according to any preceding claim wherein the dimethicone is present in an amount of from 0.5% to 5.0% by weight of the formulation.

20. A topical pharmaceutical formulation according to any preceding claim wherein the dimethicone is present in an amount of from 0.5% to 1.5% by weight of the formulation.

21. A topical pharmaceutical formulation according to any preceding claim, being an emulsion and having an oil phase, wherein the oil phase is comprised of a mixture of at least one emulsifier (emulgent) with a fat or an oil or with both a fat and an oil.

22. A topical pharmaceutical formulation according to claim 21 including a hydrophilic emulsifier together with a lipophilic emulsifier which acts as a stabiliser.

23. A topical pharmaceutical formulation according to claim 22, wherein the emulsifier (emulgent) or emulsion stabiliser used in the formulation includes cetyl alcohol, sodium lauryl sulphate, stearyl alcohol or a polyoxyethylene alkyl ether.

24. A topical pharmaceutical formulation according to claim 23, wherein the emulsifier (emulgent) or emulsion stabiliser used in the formulation includes a polyoxyethylene alkyl ether.

25. A topical pharmaceutical formulation according to any of claims 21 to 24, wherein the oil phase comprises an oil comprising white soft paraffin and/or a mineral oil.

26. A topical pharmaceutical formulation according to any preceding claim, comprising 0.5-1.5% w/w dimethicone; approximately 5% w/w aciclovir; 5-10% w/w cetostearyl alcohol; 35-45% w/w propylene glycol; 2-10% w/w mineral oil; 10-15% w/w white petrolatum; 0.5-1% w/w sodium lauryl sulphate; 0.5-2% w/w poloxamer 407 and purified water to 100% w/w.

27. Use of aciclovir, dimethicone and at least 30% w/w of a water miscible polyhydric alcohol in the preparation of a topical medicament for the treatment of herpes family viral infections;
wherein the water miscible polyhydric alcohol is propylene glycol, butane 1,3-diol or diethylene glycol monoethyl ether, and wherein the dimethicone is present in an amount of from 0.1% to 10% by weight of the medicament.

28. Use of dimethicone in decreasing the irritancy of topical oil-in-water formulations of aciclovir comprising at least 30% w/w of a water miscible polyhydric alcohol;
wherein the water miscible polyhydric alcohol is propylene glycol, butane 1,3-diol or diethylene glycol monoethyl ether, and wherein the dimethicone is present in an amount of from 0.1% to 10% by weight of the formulation.

29. The use according to claim 27 or 28, wherein the formulation or medicament contains at least 30% by weight of the water miscible polyhydric alcohol and a maximum of 50% water by weight of the formulation or medicament.

30. A method for the preparation of a topical pharmaceutical formulation, as defined in any of claims 1 to 26, which comprises mixing a combination of the aciclovir, the water miscible polyhydric alcohol and water with an oil phase and the dimethicone to form a cream emulsion.

31. A method according to claim 30 wherein the aciclovir and the water miscible polyhydric alcohol are initially incorporated wholly in an aqueous portion where they form a solution or a mixed solution/suspension, and are then emulsified with an ointment base and the dimethicone.

32. A method according to claim 30 wherein a part of an aqueous portion is formulated with an ointment base as an emulsion, and the balance of the water miscible polyhydric alcohol and aciclovir is added to and dispersed into the emulsion.

33. A method according to claim 31 or 32 comprising heating the aqueous portion and the ointment base to 40 to 80°C prior to emulsification.

34. A topical pharmaceutical formulation according to any of claims 1 to 26, preparable by a method as defined in any of claims 30 to 33.

## Patentansprüche

1. Topische pharmazeutische Formulierung, die Aciciovir, Dimethicon und mindestens 30 Gew.-% eines mit Wasser mischbaren mehrwertigen Alkohols umfasst;
wobei der mit Wasser mischbare mehrwertige Alkohol Propylenglycol, Butan-1,3-diol oder Diethylenglycolmonoethylether ist, und wobei Dimethicon in einer Menge von 0,1 bis 10 Gew.-% der Formulierung vorliegt.

2. Topische pharmazeutische Formulierung nach Anspruch 1, die eine Öl-in-Wasser-Formulierung ist.

3. Topische pharmazeutische Õl-in-Wasser-Formulierung, die eine kontinuierliche wässrige Phase und in ihr dispergiert eine Ölphase umfasst,
wobei die Formulierung Wasser, solubilisiertes Aciclovir, Dimethicon und mindestens 30 Gew.-% eines mit Wasser mischbaren mehrwertigen Alkohols umfasst;
wobei der mit Wasser mischbare mehrwertige Alkohol Propylenglycol, Butan-1,3-diol oder Diethylenglycolmonoethylether ist, und wobei Dimethicon in einer Menge von 0,1 bis 10 Gew.-% der Formulierung vorliegt.

4. Topische pharmazeutische Formulierung nach einem der Ansprüche 1 bis 3, welche mindestens 30 Gew.-% der Formulierung des mit Wasser mischbaren mehrwertigen Alkohols und maximal 50 Gew.-% der Formulierung an Wasser enthält.

5. Topische pharmazeutische Formulierung, die Wasser, Aciclovir, Dimethicon und mindestens 30 Gew.-% der Formulierung des mit Wasser mischbaren mehrwertigen Alkohols umfasst; wobei die Formulierung maximal 50 Gew.-% der Formulierung an Wasser enthält;
wobei der mit Wasser mischbare mehrwertige Alkohol Propylenglycol, Butan-1,3-diol oder Diethylenglycolmonoethylether ist, und Dimethicon in einer Menge von 0,1 Gew.-% bis 10 Gew.-% der Formulierung vorliegt.

6. Topische pharmazeutische Formulierung nach Anspruch 5, welche eine topische pharmazeutische Öl-in-Wasser-Formulierung ist.

7. Topische pharmazeutische Formulierung nach einem der Ansprüche 1 bis 6, wobei der mehrwertige Alkohol in einer oder der wässrigen Phase in einer Menge von bis zu etwa 50 Gew.-% enthalten ist.

8. Topische pharmazeutische Formulierung nach einem der Ansprüche 1 bis 7, enthaltend 0,075 bis 10 Gew.-% Aciclovir oder ein Salz davon, von 30 bis 50 Gew.-% des mit Wasser mischbaren mehrwertigen Alkohols, von 15 bis 50 Gew.-% Wasser, Dimethicon und eine Ölphase.

9. Topische pharmazeutische Formulierung nach Anspruch 8, umfassend von 0,5 bis 10 Gew.-% Aciclovir, von 30 bis 45 Gew.-% des mit Wasser mischbaren mehrwertigen Alkohols und von 20 bis 40 Gew.-% Wasser zusammen mit einer Ölphase und Dimethicon.

10. Topische pharmazeutische Formulierung nach einem der Ansprüche 1 bis 9, wobei der mit Wasser mischbare mehrwertige Alkohol Propylenglycol oder Diethylenglycolmonoethylether ist.

11. Topische pharmazeutische Formulierung nach einem der Ansprüche 1 bis 9, wobei der mit Wasser mischbare mehrwertige Alkohol Propylenglycol ist.

12. Topische pharmazeutische Formulierung nach Anspruch 11, umfassend von 35 bis 45 Gew.-% der Formulierung an Propylenglycol.

13. Topische pharmazeutische Öl-in-Wasser-Formulierung nach einem der Ansprüche 2 bis 9, wobei der mehrwertige Alkohol Propylenglycol ist.

14. Topische pharmazeutische Öl-in-Wasser-Formulierung nach Anspruch 13, wobei das Propylenglycol in einer Menge von 30 Gew.-% bis 45 Gew.-% vorhanden ist.

15. Topische pharmazeutische Öl-in-Wasser-Formulierung nach Anspruch 13, wobei das Propylenglycol in einer Menge von 30 Gew.-% bis 40 Gew.-% vorhanden ist

16. Topische pharmazeutische Formulierung nach einem der vorstehenden Ansprüche, wobei das Dimethicon in einer Menge vorhanden ist, die ausreicht, um die Reizung durch die Formulierung deutlich zu verringern.

17. Topische pharmazeutische Formulierung nach einem der vorstehenden Ansprüche, wobei Dimethicon in einer Menge von 0,5 bis 10 Gew.-% der Formulierung vorliegt.

18. Topische pharmazeutische Formulierung nach einem der vorstehenden Ansprüche, wobei Dimethicon in einer Menge von 0,1 bis 5,0 Gew.-% der Formulierung vorliegt.

19. Topische pharmazeutische Formulierung nach einem der vorstehenden Ansprüche, wobei das Dimethicon in einer Menge von 0,5 Gew.-% bis 5,0 Gew.-% der Formulierung vorliegt.

20. Topische pharmazeutische Formulierung nach einem der vorstehenden Ansprüche, wobei das Dimethicon in einer Menge von 0,5 Gew.-% bis 1,5 Gew.-% der Formulierung vorliegt.

21. Topische pharmazeutische Formulierung nach einem der vorstehenden Ansprüche, welche eine Emulsion ist und eine Ölphase aufweist, wobei die Ölphase aus einem Gemisch aus mindestens einem Emulsionsmittel (Emulgator) mit einem Fett oder Öl oder sowohl mit einem Fett und einem Öl besteht.

22. Topische pharmazeutische Formulierung nach Anspruch 21 enthaltend ein hydrophiles Emulsionsmittel zusammen mit einem lipophilen Emulsionsmittel, das als Stabilisator wirkt.

23. Topische pharmazeutische Formulierung nach Anspruch 22, wobei das in der Formulierung verwendete Emulsionsmittel (Emulgator) oder der Emulsionsstabilisator Cetylalkohol, Natriumlaurylsulfat, Stearylalkohol oder Polyoxyethylenalkylether einschließt.

24. Topische pharmazeutische Formulierung nach Anspruch 23, wobei das in der Formulierung verwendete Emulsionsmittel (Emulgator) oder der Emulsionsstabilisator einen Polyoxyethylenalkylether einschließt.

25. Topische pharmazeutische Formulierung nach einem der Ansprüche 21 bis 24, wobei die Ölphase ein Öl umfasst, welches weißes weiches Paraffin und/oder ein Mineralöl umfasst.

26. Topische pharmazeutische Formulierung nach einem der vorstehenden Ansprüche, umfassend 0,5-1,5 Gew.-% Dimethicon; ungefähr 5 Gew.-% Aciclovir; 5-10 Gew.-% Cetostearylalkohol; 35-45 Gew.-% Propylenglycol; 2-10 Gew.-% Mineralöl; 10-15 Gew,-% weißes Petrolatum; 0,5-1 Gew.-% Natriumlaurylsulfat; 0,5-2 Gew.-% Poloxamer 407 and gereinigtes Wasser zu 100 Gew.-%.

27. Verwendung von Aciclovir, Dimethicon und mindestens 30 Gew.-% eines mit Wasser mischbaren mehrwertigen Alkohols für die Herstellung eines topischen Medikaments zur Behandlung von viralen Infektionen der Herpes-Familie;
wobei der mit Wasser mischbare mehrwertige Alkohol Propylenglycol, Butan-1,3-diol oder Diethylenglycol-monoethylether ist, und wobei Dimethicon in einer Menge von 0,1 bis 10 Gew.-% des Medikaments vorliegt

28. Verwendung von Dimethicon zur Verringerung der Reizung von topischen Ölin-Wasser-Formulierungen von Aciclovir, umfassend mindestens 30 Gew.-% eines mit Wasser mischbaren mehrwertige Alkohols;
wobei der mit Wasser mischbare mehrwertige Alkohol Propylenglycol, Butan-1,3-diol oder Diethylenglycolmonoethylether ist, und wobei Dimethicon in einer Menge von 0,1 bis 10 Gew.-% der Formulierung vorliegt.

29. Verwendung nach Anspruch 27 oder 28, wobei die Formulierung oder das Medikament mindestens 30 Gew.-% des mit Wasser mischbaren mehrwertigen Alkohols und höchstens 50 Gew.-% der Formulierung oder des Medikaments an Wasser enthält.

30. Verfahren zur Herstellung einer topischen pharmazeutischen Formulierung nach einem der Ansprüche 1 bis 26, umfassend das Mischen einer Kombination von Aciclovir, des mit Wasser mischbaren mehrwertigen Alkohols und Wasser mit einer Öl-Phase und dem Dimethicon zur Bildung einer cremigen Emulsion.

31. Verfahren nach Anspruch 30, wobei das Aciclovir und der mit Wasser mischbare mehrwertige Alkohol zunächst gänzlich in einen wässrigen Anteil inkorporiert werden, wo sie eine Lösung oder eine gemischte Lösung/Suspension bilden, und dann mit einer Salbengrundlage und dem Dimethicon emulgiert werden.

32. Verfahren nach Anspruch 30, wobei ein Teil eines wässrigen Anteils mit einer Salbengrundlage als Emulsion formuliert wird, und der Rest an mit Wasser mischbaren mehrwertigen Alkohols und das Acidovir zu der Emulsion gegeben und darin verteilt wird.

33. Verfahren nach Anspruch 31 und 32, umfassend das Erhitzen des wässrigen Anteils und der Salbengrundlage auf 40 bis 80°C vor dem Emulgieren.

34. Topische pharmazeutische Formulierung nach einem der Ansprüche 1 bis 26, die mit einem Verfahren wie in einem der Ansprüche 30 bis 33 definiert herstellbar ist.

## Revendications

1. Formulation pharmaceutique topique comprenant de l'aciclovir, de la diméthicone et au moins 30 % en poids d'un polyol miscible à l'eau ;
dans laquelle le polyol miscible à l'eau est le propylèneglycol, le butane 1,3-diol ou l'éther monoéthylique du diéthylèneglycol, et dans laquelle la diméthicone est présente en une quantité allant de 0,1 % à 10 % en poids de la formulation.

2. Formulation pharmaceutique topique selon la revendication 1 qui est une formulation huile dans l'eau.

3. Formulation pharmaceutique topique huile dans l'eau comprenant une phase aqueuse continue et, dispersée dedans, une phase huileuse, la formulation comprenant de l'eau, de l'aciclovir solubilisé, de la diméthicone et au moins 30 % en poids d'un polyol miscible à l'eau ;
dans laquelle le polyol miscible à l'eau est le propylèneglycol, le butane 1,3-diol ou l'éther monoéthylique du diéthylèneglycol, et dans laquelle la diméthicone est présente en une quantité allant de 0,1 % à 10 % en poids de la formulation.

4. Formulation pharmaceutique topique selon l'une quelconque des revendications 1 à 3 contenant au moins 30 % en poids du polyol miscible à l'eau et un maximum de 50 % d'eau en poids de la formulation.

5. Formulation pharmaceutique topique comprenant de l'eau, de l'aciclovir, de la diméthicone et au moins 30 % d'un polyol miscible à l'eau en poids de la formulation ;
dans laquelle la formulation contient un maximum de 50 % d'eau en poids de la formulation, dans laquelle le polyol miscible à l'eau est le propylèneglycol, le butane 1,3-diol ou l'éther monoéthylique du diéthylèneglycol, et dans laquelle la diméthicone est présente en une quantité allant de 0,1 % à 10 % en poids de la formulation.

6. Formulation pharmaceutique topique selon la revendication 5 qui est une formulation pharmaceutique topique huile dans l'eau.

7. Formulation pharmaceutique topique selon l'une quelconque des revendications 1 à 6 dans laquelle le polyol est présent dans une ou la phase aqueuse en une quantité allant jusqu'à environ 50 % en poids.

8. Formulation pharmaceutique topique selon l'une quelconque des revendications 1 à 7 contenant de 0,075 % à 10 % en poids d'aciclovir ou d'un sel de celui-ci, de 30 % à 50 % en poids du polyol miscible à l'eau, de 15 % à 50 % en poids d'eau, de la diméthicone et une phase huileuse.

9. Formulation pharmaceutique topique selon la revendication 8 comprenant de 0,5 % à 10 % en poids d'aciclovir, de 30 % à 45 % en poids du polyol miscible à l'eau et de 20 % à 40 % en poids d'eau, le tout avec une phase huileuse et de la diméthicone.

10. Formulation pharmaceutique topique selon l'une quelconque des revendications 1 à 9, dans laquelle le polyol miscible à l'eau est le propylèneglycol ou l'éther monoéthylique du diéthylèneglycol.

11. Formulation pharmaceutique topique selon l'une quelconque des revendications 1 à 9, dans laquelle le polyol miscible à l'eau est le propylèneglycol.

12. Formulation pharmaceutique topique selon la revendication 11 comprenant de 35 % à 45 % de propylèneglycol en poids de la formulation.

13. Formulation pharmaceutique topique huile dans l'eau selon l'une quelconque des revendications 2 à 9, dans laquelle le polyol est le propylèneglycol.

14. Formulation pharmaceutique topique huile dans l'eau selon la revendication 13 dans laquelle le propylèneglycol est présent en une quantité allant de 30 % à 45 % en poids.

15. Formulation pharmaceutique topique huile dans l'eau selon la revendication 13 dans laquelle le propylèneglycol est présent en une quantité allant de 30 % à 40 % en poids.

16. Formulation pharmaceutique topique selon l'une quelconque des revendications précédentes dans laquelle la diméthicone est présente en une quantité suffisante pour réduire de façon significative le caractère irritant de la formulation.

17. Formulation pharmaceutique topique selon l'une quelconque des revendications précédentes dans laquelle la diméthicone est présente en une quantité allant de 0,5 % à 10 % en poids de la formulation.

18. Formulation pharmaceutique topique selon l'une quelconque des revendications précédentes dans laquelle la diméthicone est présente en une quantité allant de 0,1 % à 5,0 % en poids de la formulation.

19. Formulation pharmaceutique topique selon l'une quelconque des revendications précédentes dans laquelle la diméthicone est présente en une quantité allant de 0,5 % à 5,0 % en poids de la formulation.

20. Formulation pharmaceutique topique selon l'une quelconque des revendications précédentes dans laquelle la diméthicone est présente en une quantité allant de 0,5 % à 1,5 % en poids de la formulation.

21. Formulation pharmaceutique topique selon l'une quelconque des revendications précédentes, étant une émulsion et ayant une phase huileuse, dans laquelle la phase huileuse est constituée d'un mélange d'au moins un émulsifiant (émulgent) avec un corps gras ou une huile, ou avec à la fois un corps gras et une huile.

22. Formulation pharmaceutique topique selon la revendication 21 comprenant un émulsifiant hydrophile et un émulsifiant lipophile qui agit comme stabilisateur.

23. Formulation pharmaceutique topique selon la revendication 22, dans laquelle l'émulsifiant (émulgent) ou stabilisateur d'émulsion utilisé dans la formulation comprend de l'alcool cétylique, du sulfate de lauryle sodique, de l'alcool stéarique ou un éther de polyoxyéthylène d'alkyle.

24. Formulation pharmaceutique topique selon la revendication 23, dans laquelle l'émulsifiant (émulgent) ou stabilisateur d'émulsion utilisé dans la formulation comprend un éther de polyoxyéthylène d'alkyle.

25. Formulation pharmaceutique topique selon l'une quelconque des revendications 21 à 24, dans laquelle la phase huileuse comprend une huile composée de paraffine molle blanche et/ou d'une huile minérale.

26. Formulation pharmaceutique topique selon l'une quelconque des revendications précédentes, comprenant de 0,5 à 1,5 % en poids de diméthicone ; environ 5 % en poids d'aciclovir ; de 5 à 10 % en poids d'alcool cétostéarylique ; de 35 à 45 % en poids de propylèneglycol ; de 2 à 10 % en poids d'huile minérale ; de 10 à 15 % en poids de vaseline blanche ; de 0,5 à 1 % en poids de sulfate de lauryle sodique ; de 0,5 à 2 % en poids de poloxamère 407 et d'eau purifiée jusqu'à 100 % du poids.

27. Utilisation d'aciclovir, de diméthicone et d'au moins 30 % en poids d'un polyol miscible à l'eau pour la préparation d'un médicament topique pour le traitement des infections virales de la famille de l'herpès ; dans laquelle le polyol miscible à l'eau est le propylèneglycol, le butane 1,3-diol ou l'éther monoéthylique du diéthylèneglycol, et dans laquelle la diméthicone est présente en une quantité allant de 0,1 % à 10 % en poids du médicament.

28. Utilisation de la diméthicone pour réduire le caractère irritant des formulations topiques huile dans l'eau d'aciclovir comprenant au moins 30 % en poids d'un polyol miscible à l'eau ; dans laquelle le polyol miscible à l'eau est le propylèneglycol, butane 1,3-diol ou l'éther monoéthylique du diéthylèneglycol, et dans laquelle la diméthicone est présente en une quantité allant de 0,1 % à 10 % en poids de la formulation.

29. Utilisation selon la revendication 27 ou 28, dans laquelle la formulation ou le médicament contient au moins 30 % en poids du polyol miscible à l'eau et un maximum de 50 % d'eau en poids de la formulation ou du médicament.

30. Procédé de préparation d'une formulation pharmaceutique topique telle que définie selon l'une quelconque des revendications 1 à 26, comprenant le mélange d'une combinaison de l'aciclovir, du polyol miscible à l'eau et d'eau avec une phase huileuse et la diméthicone pour former une émulsion crème.

31. Procédé selon la revendication 30 dans lequel l'aciclovir et le polyol miscible à l'eau sont initialement incorporés entièrement dans une portion aqueuse où ils forment une solution ou un mélange de suspension/solution, et sont ensuite émulsifiés avec un excipient pour pommade et la diméthicone.

32. Procédé selon la revendication 30 dans lequel une partie d'une portion aqueuse est formulée avec un excipient pour pommade en tant qu'émulsion, et le reste formé par le polyol miscible à l'eau et l'aciclovir est ajouté et dispersé dans l'émulsion.

33. Procédé selon la revendication 31 ou 32 comprenant le chauffage de la portion aqueuse et de l'excipient pour pommade de 40 à 80°C avant émulsification.

34. Formulation pharmaceutique topique selon l'une quelconque des revendications 1 à 26, pouvant être préparée par un procédé défini selon l'une quelconque des revendications 30 à 33.
